# EUROPEAN PATENT APPLICATION

(11) **EP 2 266 962 A1**
(43) Date of publication of application: **29.12.2010**
(21) Application number: 09719079.7
(22) Date of filing: 05.03.2009
(51) Int. Cl.: C07D 239/88, C07D 239/86, C07D 239/90, C07D 239/91, C07D 471/04, C07D 487/04, A61K 31/517, A61K 31/519, A61P 3/00, A61P 3/04, A61P 3/06, A61P 3/10, A61P 9/00, A61P 9/10

(54) **THE COMPOUNDS AS THE ESTROGEN RELATED RECEPTORS MODULATORS AND THE USES THEREOF**

(30) Priority: 13.03.2008 CN 200810026782
(71) Applicant: Guangzhou Institute Of Biomedicine And Health, Chinese Academy Of Sciences, Guangdong (CN)
(72) Inventor: DING, Ke, Guangzhou Guangdong 510663 (CN); WONG, Chiwai, Guangzhou Guangdong 510663 (CN); KANG, Zhanfang, Guangzhou Guangdong 510663 (CN); ZHOU, Xi, Guangzhou Guangdong 510663 (CN)
(74) Representative: Brown, David Leslie
(86) International application number: PCT/CN2009/000234
(87) International publication number: WO 2009/111943

(57) **Abstract**

The compounds according to formula (I), their pharmaceutically acceptable acid or base addition salts, and the uses thereof is disclosed. These compounds and their pharmaceutically acceptable acid or base addition salts can be used for the preparing a medicament for modulating estrogen related receptor (ERR), and treating metabolic diseases, such as high blood fat, fatty liver, hyperglycemia, diabetes, obesity, etc. The definition of the groups of the formula is defined as the description.

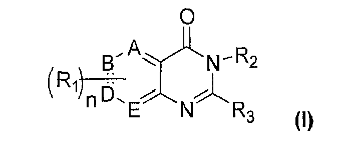

## Description

### FIELD OF THE INVENTION

This invention relates to the compounds as the estrogen related receptors modulators and the uses thereof.

### BACKGROUND OF THE INVENTION

The increasing incidences of metabolic diseases including obesity, diabetes, dyslipidemia, hypertension, and atherosclerosis, are leading to higher risks of heart diseases, a leading cause of mortality worldwide. The healthcare cost associated with treatment is putting major burdens on the healthcare systems of developed as well as developing countries. Therefore, identifying novel targets and pharmacologic agents to treat and/or prevent these disorders are of high priorities.

Both type 1 (insulin-dependent diabetes mellitus, IDDM) and type 2 (noninsulin-dependent diabetes mellitus, NIDDM) diabetes are characterized by elevated levels of plasma glucose (hyperglycemia) in the fasting state or after administration of glucose during an oral glucose tolerance test. Insulin is the hormone that regulates glucose utilization by stimulating glucose and lipid metabolism in the main insulin-sensitive tissues including muscle, liver and adipose tissues. Inappropriate regulation of energy metabolism in these tissues accounts for most of the alterations in glucose homeostasis seen in patients with type 2 diabetes. In addition, patients having type 2 diabetes often have hyperinsulinemia (elevated plasma insulin levels). Insulin resistance, which means a resistance to the effect of insulin, plays an early role in the pathogenesis of type 2 diabetes.

Skeletal muscle and liver are the two key insulin-responsive organs responsible for maintaining normal glucose homeostasis. Mitochondrial dysfunction has been closely associated with skeletal muscle insulin resistance in several studies. In skeletal muscle of human type II diabetics, the expression levels of mitochondrial oxidative phosphorylation (OXPHOS) genes are reduced. The OXPHOS genes that are dysregulated in type II diabetic patients are under the transcriptional control of peroxisome proliferator-activated receptor .γ coactivator-1α (PGC-1α). Estrogen related receptor α (ERRα) is expressed in tissues with a high capacity for β-oxidation of fatty acids including the heart, kidneys, brown adipose tissue and skeletal muscle. ERRα is primarily thought to regulate energy homeostasis through interacting with PGC-1α and coordinately control the transcription of genes in the oxidative phosphorylation pathway. ERRα has also been shown to modulate fatty acid and glucose utilization through directly regulating the expression of phosphoenolpyruvate carboxykinase (PEPCK), medium chain acyl dehydrogenase (MCAD), and pyruvate dehydrogenase kinase 4 (PDK4). Interestingly, in certain forms of cardiac hypertrophy and heart failure, the heart uses glucose instead of fatty acid as an energy source.

Reduction of estrogen levels in post-menopausal results in an increase of bone loss leading to osteoporosis. Over-expression of ERRα in osteoblasts increases bone nodule formation, while reducing the expression by anti-sense results in a decrease of bone nodule formation. Therefore, compounds that enhance the activity of estrogen related receptors (ERRα, β, and γ, etc.) activity may have an anabolic effect for the regeneration of bone density. Conversely, with respect to bone diseases that are a result of abnormal bone growth, compounds that will interact with estrogen related receptors (ERRα, β, and γ, etc.) and decrease its biological activity may provide a benefit for the treatment of these diseases by retarding bone growth.

Although estrogen related receptors alpha, beta and gamma (ERRα, ERRβ and ERRγ) are considered to be orphan nuclear hormone receptors that display constitutively active transcriptional activities, synthetic phenolic acyl hydrazones have recently been demonstrated to be selective ERRβ and ERRγ agonists through binding to the C-terminally located ligand binding domain (LBD) and activating its function. However, no definitive ERRα agonist has been identified so far that would improve insulin resistance through enhancing the function of PGC1α.

### SUMMARY OF THE INVENTION

It is an object of the present invention to provide compounds as the estrogen related receptors modulators.

To achieve the above object, the technical solution is as followings: compounds having formula I or their pharmaceutical acceptable salts or stereo isomers: 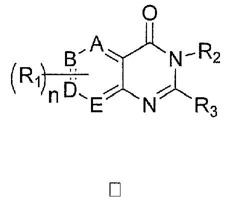 wherein
A, B, D, and E are independently selected as CH or N;
n is 0, 1 or 2;
in the following, a is 0 or 1, b is 0 or 1;
R₁ is selected from the following:
1)H;
2) halide (F, Cl, Br, I);
3)OH;
4) NO₂;
5) CO₂H;
6)(C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₇;
16) SO₂C₁∼C₈ alkyl;
17) (C=O)ₐO_{b}C₀∼C₈-NR₆R₅;
the alkyl, aryl, alkenyl, alkynyl, cycloalkyl or heterocycle groups mentioned above can be substituted by 0, 1 or more than 1 substituted group independently selected from R₄;
R₂ is selected from the following:
1) H;
2) C₁∼C₈ alkyl;
3) aryl;
4) C₃∼C₈ alkenyl;
5) C₃∼C₈ alkynyl;
6) C₁∼C₈ fluoroalkyl;
7) C₁∼C₆ aryl alkyl;
8) C₃∼C₆ cycloalkyl;
9) heterocycle;
the alkyl, aryl, alkenyl, alkynyl, cycloalkyl or heterocycle groups mentioned above can be substituted by 0, 1 or more than 1 substituted group independently selected from R₄;
R₃ is selected from the following:
1) (C=O)ₐO_{b}C₁∼C₈ alkyl;
2) (C=O)ₐO_{b} aryl;
3) (C=O)ₐO_{b}C₂∼C₈ alkenyl;
4) (C=O)ₐO_{b}C₂∼C₈ alkynyl;
5) O_{b}C₁∼C₈ fluoroalkyl;
6) (C=O)ₐNR₆R₅;
7) (C=O)ₐO_{b}C₃∼C₈ cycloalkyl;
8) (C=O)ₐO_{b} heterocycle;
9) (C=O)ₐO_{b}C₃∼C₆ cycloalkyl;
the alkyl, aryl, alkenyl, alkynyl, cycloalkyl or heterocycle groups mentioned above can be substituted by 0, 1 or more than 1 substituted group independently selected from R₄;
R₄ is selected from the following:
1) H;
2) halide (F, Cl, Br, I);
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₇;
16) SO₂C₁∼C₈ alkyl;
the alkyl, aryl, alkenyl, alkynyl, cycloalkyl or heterocycle groups mentioned above can be substituted by no more than 3 substituted group independently selected from R₅, OH, (C₁∼C₆) alkoxy, halide, COOH, CN, O(C=O)C₁∼C₆ alkyl, or NR₅R₆ groups;
R₅ and R₆ are independently selected from:
1) H;
2) (C=O)ₐO_{b}C₁∼C₈ alkyl;
3) (C=O)ₐO_{b} aryl;
4) (C=O)ₐO_{b}C₂∼C₈ alkenyl;
5) (C=O)ₐO_{b}C₂∼C₈ alkynyl;
6) O_{b}C₁∼C₈ fluoroalkyl;
7) (C=O)ₐO_{b}C₃∼C₈ cycloalkyl;
8) (C=O)ₐO_{b} heterocycle;
9) SO₂ C₁∼C₈ alkyl;
10) (C=O)ₐO_{b}C₀∼C₈-NR₆R₅;
the alkyl, aryl, alkenyl, alkynyl, cycloalkyl or heterocycle groups mentioned above can be substituted by no more than 3 substituted group independently selected from R₆. R₆ and R₅ may form a 4-7-atom ring, or a bicyclic ring in which each ring is formed by 4∼7-atoms. The ring or bicyclic ring comprises 1, 2, or 3 hetero atoms selected from N, O and S atom. The ring or bicyclic ring can be substituted by one or more substituted groups independently selected from R₅.

In another embodiment, the invention relates to compounds having formula II and their pharmaceutical acceptable salts and stereo isomers: 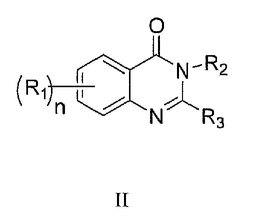 wherein, R₁∼R₃ and n are defined as that in formula I.

In other embodiments, the invention relates to compounds having formula III∼VI and their pharmaceutical acceptable salts and stereo isomers: 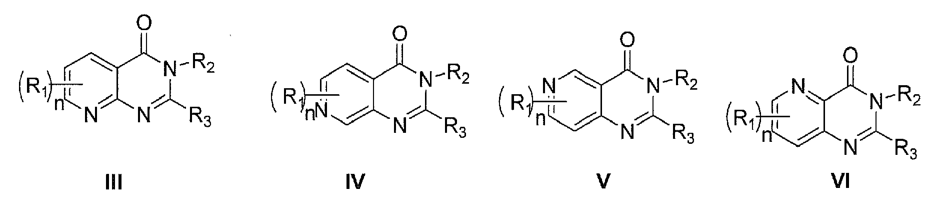 wherein, R1∼R3 and n are defined as that in formula II.

In another embodiment, the invention relates to compounds having formula VII and their pharmaceutical acceptable salts and stereo isomers: 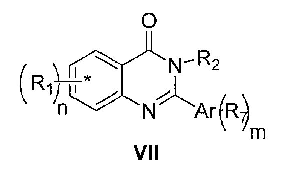 wherein,
in the following, a is 0 or 1, b is 0 or 1;
m and n are independently selected as 0, 1 or 2;
Ar is an aromatic ring containing or without containing hetero atoms such as N, S or O.
R₁ and R₇ are independently selected from the following:
1) H;
2) halide (F, Cl, Br, I);
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₅;
16) SO₂C₁∼C₈ alkyl;
17) (C=O)ₐO_{b}C₀∼C₈-NR₆R₅;
the alkyl, aryl, alkenyl, alkynyl, cycloalkyl or heterocycle group metioned above can be substituted by 0, 1 or more than 1 substituted group independently selected from R₄;
R₂ is selected from the following:
1) H;
2) C₁∼C₈ alkyl;
3) aryl;
4) C₃∼C₈ alkenyl;
5) C₃∼C₈ alkynyl;
6) C₁∼C₈ fluoroalkyl;
7) C₁∼C₆ aryl alkyl;
8) C₃∼C₆ cycloalkyl;
9) heterocycle;
the alkyl, aryl, alkenyl, alkynyl, cycloalkyl or heterocycle groups metioned above can be substituted by 0, 1 or more than 1 substituted group independently selected from R₄;
R₄ is selected from the following:
1) H;
2) halide (F, Cl, Br, I);
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₅;
16) SO₂C₁∼C₈ alkyl;
the alkyl, aryl, alkenyl, alkynyl, cycloalkyl or heterocycle group metioned above can be substituted by no more than 3 substituted group independently selected from R₅, OH, (C₁∼C₆) alkoxy, halide, COOH, CN, O(C=O)C₁∼C₆ alkyl, or NR₅R₆ groups;
R₅ and R₆ are independently selected from the following:
1) H;
2) (C=O)ₐO_{b}C₁∼C₈ alkyl;
3) (C=O)ₐO_{b} aryl;
4) (C=O)ₐO_{b}C₂∼C₈ alkenyl;
5) O_{b}C₁∼C₈ fluoroalkyl;
6) (C=O)ₐO_{b}C₃∼C₈ cycloalkyl;
7) (C=O)ₐO_{b} heterocycle;
8) SO₂C₁∼C₈ alkyl;
9) (C=O)ₐO_{b}C₀∼C₈-NR₆R₅;
the alkyl, aryl, alkenyl, alkynyl, cycloalkyl or heterocycle group metioned above can be substituted by no more than 3 substituted group independently selected from R₆. R₆ and R₅ may form a 4∼7-atom ring, or a bicyclic ring in which each ring is formed by 4∼7-atoms; the ring or bicyclic ring comprises 1, 2, or 3 hetero atoms selected from N, O or S atom; the ring or bicyclic ring can be substituted by zero or one or more substituted groups independently selected from R₅.

In another embodiment, the invention relates to compounds having formula VIII and their pharmaceutical acceptable salts and stereo isomers: 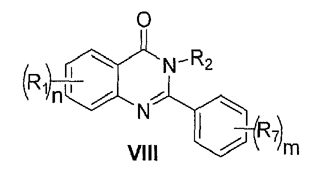 wherein, R₁, R₂, R₇, m and n are defined as that in formula VII.

The invention also relates to a pharmaceutical composition containing any one of the compounds mentioned above or their pharmaceutically acceptable salts or pro-drugs thereof. The pharmaceutical composition can be used as a new class of therapeutics for the treatment of metabolic diseases.

The present invention relates to compounds mentioned above and their pharmaceutical acceptable salts which function as modulators of estrogen-related receptors (ERRα, β, and γ, etc.) and their use as a new class of therapeutics for the treatment of metabolic diseases.

Preferably, the metabolic diseases includes: (1) Type II diabetes; (2) hyperglycemia; (3) reduced glucose tolerance; (4) insulin resistance; (5) obesity; (6) abnormal fat metabolism; (7) dyslipidemia; (8) hyperlipidemia; (9) hypertriglyceridemia; (10) hypercholesterolemia; (11) low levels of HDL; (12) high levels of LDL; (13) atherosclerosis; (14) vascular restenosis; (15) central obesity; (16) metabolic syndrome; (17) fatty liver.

The present invention relates to compounds represented by Formula I, which are agonists of estrogen-related receptors (ERRα, β, and γ, etc). The invention also relate to the use of compounds of the invention to treat a subject suffering from or diagnosed with metabolic diseases like Type II diabetes and associated dyslipidemia, hyperlipidemia, hypertriglyceridemia, hypercholesterolemia, obesity and fatty liver.

The compounds of the present invention which agonize the functions of ERRα and its interacting partner PGC-1α will alleviate the extent of insulin resistance, improve glucose homeostasis in diabetic patients and restore insulin sensitivity. These compounds may reduce blood glucose levels and diabetic serum marker hemoglobin A1c glycosylation level. The present invention contemplates that ERRα agonists may enhance the therapeutic effects of current and developing insulin sensitizers and insulin secertagogues when used in combination.

### Brief Description of the Drawings

Fig. 1 is a diagram showing the effect of DK3 on the activity of ERRα;
Fig. 2 is a diagram showing the effect of DK3 on the activity of ERRα/β/γ;
Fig. 3 is a diagram showing the effect of DK compounds on the activity of ERRα;
Fig. 4 is a diagram showing the effect of DK3 on the reporter gene expression of the promoter PGC1α driven by ERRα;
Fig. 5 is a diagram showing the effect of DK1 compound on the absorption of glucose;
Fig. 6 is a diagram showing the effect of compounds DK1 and DK3 on the glucose tolerance;
Fig. 7 is a diagram showing the effect of compounds DK1 and DK3 on the weight of animal liver;
Fig. 8 is an image showing the effect of DK1 and DK3 on improving the fatty liver of small rats induced by feeding with high-fat diet.

### DETAILED DESCRIPTION OF THE PREFERRED EMBODIMENT

The compounds related to the invention could have chiral center, chiral axis or chiral surface.They may have racemate. All the stero isomers, racemate mixtures and other isomers are included in the invention. The compounds related to the invention may have tautomers. Although there is only one taumoter is described, the invention included all the possible taumoters. For example, the following claimed compound A should be considered as including the isomer B and the mixture of them, and vice versa.

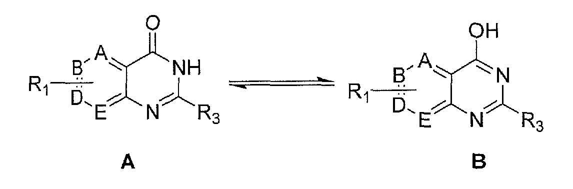

When any variable (for example, R3, R4, R5, R6, R7, etc.) appear in any component more than once, its definition of each place where it occurs independent of the other definition of each place where it occurs. Similarly, it is allowed that all the substituent and variables could be combined, if the combination is feasible. If a line was drawn from a substituent into a ring, it means that the substituent could be at any feasible position in the ring. If a polycyclic aromatic ring system is applied, it means that the substituent is only connected to the appropriate carbon atoms in the adjacent ring. If the substituents themselves being substituted by more than one group, these groups should be sunstituted in the same or different carbon atoms. The phrase "one or more optional substituents " are considered the phrase "at least one optional substituent was replaced ". In this case the implementation will be substituted with 0-3 substituents.

In this invention, the term "alkyl" and "sub-alkyl" means a ranched-chain or straight chain alkyl group with certain number of carbon atoms. For example, the "C1-C8" in "C1-C8 alkyl" is defined to straight-chain or branched-chain alkyl group with 1,2,3,4,5,6,7 or 8 carbon atoms. "C1-C8 alkyl" includes methyl, ethyl, n-propyl, isopropyl, n-butyl, tert-butyl, isobutyl, pentyl, hexyl, heptyl-, octyl, etc. The term "cycloalkyl" refers to a specific single saturated ring alkyl with certain number of carbon atoms. For examples, "cycloalkyl" includes cyclopropyl-, methyl - cyclopropyl-, 2, 2 - dimethyl - cyclobutyl, 2 - ethyl - cyclopentadienyl-, cyclohexyl, etc.

"Alkoxy" means a sunstituent connecting certain number of carbon atoms of the cyclic alkyl or noncyclic alkyl group through oxygen atom.

If the number of carbon atoms is not given, the term "alkenyl" means a non-aromatic hydrocarbon-based straight chain, branched-chain or cyclic ring containin 2 -8 carbon atoms and at least one carbon - carbon double bon. For example, "C2-C6 alkenyl" means an alkenyl group containing 2 - 6 carbon atoms. Alkenyl group includes vinyl, propenyl, butenyl, 2 - methyl-butenyl-and alkenyl cyclohexyl group etc.

The term "alkynyl" means a non-aromatic straight chain, branch chain, or cyclic ring containing 2 - 8 carbon atoms and at least one carbon-carbon triple bond. "C2-C6 alkynyl" means an alkynyl group containing 2 - 6 carbon atoms.

In some cases, the substituent can be defined by using certain number of carbon atoms including 0, Such as (C0 - C6) alkenyl - aryl. If ary group is considered as phenyl, this definition includes phenyl, -CH₂Ph, -CH₂CH₂Ph, -CH(CH₃)CH₂CH(CH₃)Ph, etc.

In this invention, "aryl" is a stable monocyclic ring with up to seven atoms or a stable bicyclic ring in which each ring contains up to seven carbon atoms. At least one of the rings is aromatic ring. Aryl groups include Phenyl, naphthyl, tetrahydro-naphthyl, indenyl, and biphenyl groups.

In this invention, "hetero aryl group" is a stable monocyclic ring with up to seven atoms or a stable bicyclic ring in which each ring contains up to seven carbon atoms. At least one of the rings is aromatic ring containing 1∼4 O, N or S atoms. Hetero aryl groups include but not limited to: Acridinyl, carbazolyl, morpholinyl, quinolinyl, phenanthrolinyl, pyrrazolyl, Indolyl, benzotriazolyl, furanyl, thienyl, benzo thienyl, benzo furanyl, quinolyl, isoquinolinyl, oxazolyl, isoxazolyl, pyrazinyl, pyridazinyl, pyridyl, pyrimidinyl, pyrrolyl, tetrahydro-quinolinyl, etc.

"Heterocyclyl" is an aromatic or nonaromatic ring containing 5 ∼ 1 atoms, in which contains 1∼4 htetero atoms such as O, N, S. "Heterocycle"includes the hetero aromatic ring as mentioned above and also includes dihydro and tetrahydro analogs. "Heterocycles" include but not limited to: benzimidazolyl, benzo furyl, benzopyranyl, benzo pyrazolyl, benzotriazolyl, benzo thienyl, benzoxazolyl, carbazolyl, carbolinyl, miso-phenanthrolinyl, furyl, imidazolyl, dihydro- indolyl, indolyl, indolazinyl, indazolyl, furans isobenzofuranyl, isoquinolinyl, isothiazolyl, isoxazolyl, Chennai pyridyl, oxadiazolyl, oxazolyl, oxazolinyl, isoxazole morpholinyl, oxetanyl, pyranyl, pyrazinyl, pyrazolyl, pyridazinyl, pyridazinyl, pyridyl, pyrimidinyl, pyrrolyl, quinazolinyl, quinolyl, tetrahydro pyranyl, tetrazolyl, pyridyl tetrazolyl, thiadiazolyl, thiazolyl, thienyl, triazolyl, I, 4 - alkyl-dioxinyl, hexallydroazepinyl, piperazinyl, piperidinyl, pyridine -2 - keto, alkyl pyrrolidinyl, morpholinyl, thiomorpholinyl, dihydro-benzimidazolyl, dihydro-benzo furyl, benzo-dihydro-thienyl, dihydro-benzoxazolyl, dihydro-furyl, dihydro-benzimidazolyl, dihydro-indolyl, dihydro-isoxazolyl, dihydro-iso thiazolyl, dihydro-oxadiazolyl, dihydro-oxazolyl, dihydro-pyrazinyl, dihydro-pyrazolyl, dihydro pyridyl, dihydro-pyrimidinyl, dihydro-pyrrolyl, dihydrofolate quinolyl, tetrazolyl dihydro, dihydro-thiadiazolyl, dihydro thiazolyl, dihydro thienyl, dihydro-triazolyl, methylene dioxy benzophenone acyl and their N-oxides, etc.

In an embodiment, heterocyclyl is selected from benzimidazolyl, imidazolyl, 2 - imidazoline ketone, indole-based, isoquinolinyl, morpholinyl, piperidinyl, piperazinyl, pyridyl, alkyl pyrrole, 2 - piperidine ketone, 2 - pyrimidine ketone, 2 - pyrrolidone, quinolyl, tetrahydrofuranyl, tetrahydro isoquinolinyl and thienyl.

As it can be easily understood, halides used in the invention include chlorine, fluorine, bromine and iodine.

Unless specially mentioned, alkyl, alkenyl, alkynyl cycloalkyl, aryl, hetero aryl, heterocyclic groups can be substituted or not be substituted. For example, C₁ - C₆ alkyl group can be substituted by 1, 2, or 3 substitutents selected from OH, oxo, halides, alkoxyl, dialkylamino, or hetero cyclic ring such as morpholinyl, piperidinyl groups. In this way, if one substitutent is oxo and the other substitutent is OH, then it includes - (C=O) CH₂ CH (OH) CH₃: , -C=O) OH, -CH₂ (OH) CH₂CH (O), etc.

In an embodiment, R₆ and R₅ may form a mono ring containing 4∼7 atoms or a bicyclic ring in which each ring comprises 4∼7 atoms through the N atom which connects R₅ and R₆. The mono ring or bicyclic ring may further comprises 1∼2 hetero atoms selected as N, O, S. The mono ring or bicyclic ring can also be substituted by 1 or more sunstituents selected as R₅. The hetero cyclic rings formed includebut not limit to the following heterocycles:

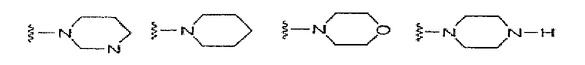

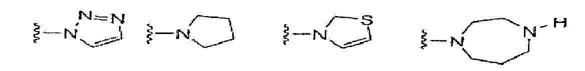

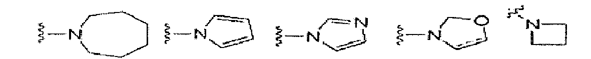

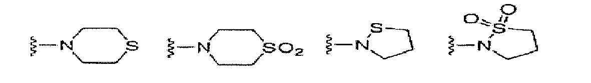

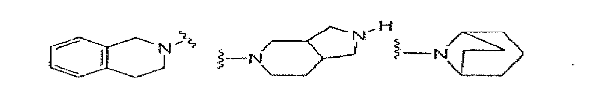

In an embodiment, R₁ is selected as halogen, hydroxy, or (C₁ - C₆) alkyl, alkoxy.

In an embodiment, R₂ is selected as H, alkyl, or alkyl group substituted by R₅.

In an embodiment, R₃ is selected as phenyl, naphthyl, cycloalkyl or pyrazolyl group which is optimally substituted by 1∼3 R₄ groups. In another embodiment, R₃ is selected as phenyl which is optimally substituted by 1∼3 R₄ groups.

In an embodiment, a is 0, and b is 1. In another embodiment, a is 0, and b is 0.

The invention relates to the free forms of compounds with formula I. It also relates to the pharmaceutical acceptable salts or stero isomers of formula I. In one embodiment, the special examples in the invetion are the protonated salts of amines. The "free form" means amines which do not form salts with acids. "Pharmaceutical acceptable salts" include all the salt forms of Formula I.

"Pharmaceutical acceptable salts"in the invention mean the salts formed by the basic compounds in the invetion with normal nontoxicic organic acids and inorganic acids. The acids include but not limited to: hydrochloric acid, hydrobromic acid, sulfuric acid, sulfamic acid, phosphoric acid, nitric acid, acetic acid, propionic acid, succinic acid, glycolic acid, stearic acid, lactic acid, malic acid, tartaric acid, lemon acid, ascorbic acid, bashing acid, maleic acid, hydroxy-maleic acid, phenylacetic acid, glutamic acid, benzoic acid, salicylic acid, sulfanilic acid, 2-acetoxy-benzoic acid 1, p-toluenesulfonic acid, methanesulfonic acid, ethane disulfonic, oxalic acid, hydroxyethyl sulfonic acid, trifluoroacetic acid etc.

If the related compounds are acids, "pharmaceutical acceptable salts" mean the salts formed by the acidic compounds in the invention with normal nontoxicic organic bases or inorganic bases. The salts formed by acidic compounds with inorganic bases include but not limited to: aluminum, ammonium, calcium, copper, iron salt, ferrous salt, lithium salt, magnesium salt, manganese salt, manganese sub-salt, potassium, sodium, zinc etc. Especially preferred ammonium salt, calcium, magnesium, potassium and sodium. The organic bases include but not limited to: primary amine, secondary amine , tertiary amine salts, substituted amines including naturally occurring substituted amines, cyclic amines and basic ion exchange resins such as arginine, betaine, caffeine, choline, N, N '- dibenzyl-ethylenediamine, diethylamine, 1, 2 diethyl amino alcohol, dimethyl amino ethanol, amino-ethanol, ethanolamine, ethylenediamine, N-ethyl morpholine, N-ethyl piperidine, glucose amine, glucosamine, histidine, hydroxyproline cobalt amine, isopropyl amine, lysine, methyl-glucosamine, morpholine, piperazine, piperidine, polyamine resins, procaine, purine, pentoxifylline, triethyl amine, trimethyl amine, tripropyl amine, ammonia hybroxybutyric triol, etc.

The related compounds can be prepared by using the following method. Of note, the synthetic scheme only outlines the examples. The related compounds may have more different substituents and can be made by other methods.

As shown in the scheme A, compound VIII was synthesized through two steps by using 2-aminobenzoic acid derivatives as the starting material. Scheme B illustrate another route to synthesize compound VIII.

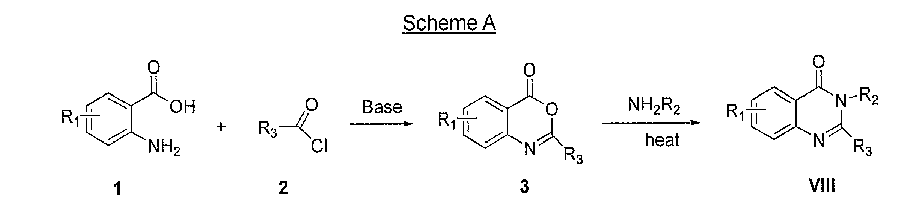

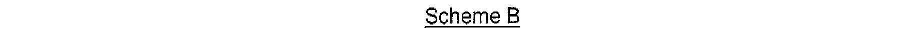

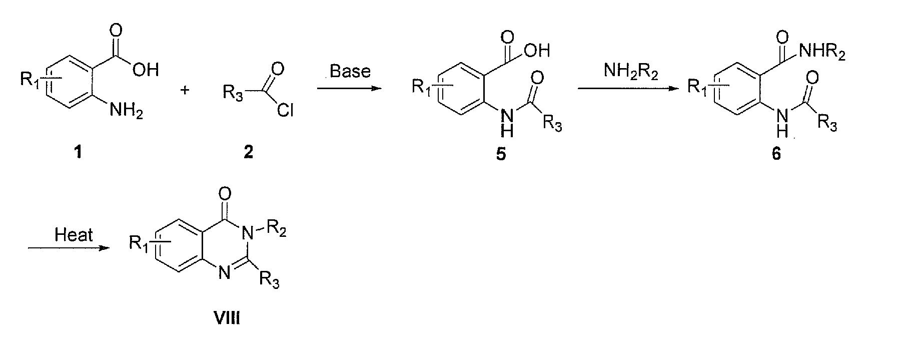

The present invention contemplates that compounds which agonize the function of ERRs, especially agonists or partial agonists of ERRα. Certain compounds can functionally stimulate the functions of both ERRα and ERRβ and consider ERRα/β dual agonists. Certain compounds can functionally stimulate the functions of both ERRα and ERRγ and consider ERRα/γ dual agonists. Certain compounds can functionally stimulate the functions of both ERRα ERRβ and ERRγ and consider ERRα/β/γ pan-agonists. The invention also relate to the use of compounds of the invention to treat a subject suffering from or diagnosed with a disease, disorder, or medical condition mediated by estrogen-related receptors.

The present invention contemplates that compounds which agonize the functions of ERRα and its interacting partner PGC-1α will alleviate the extent of insulin resistance, improve glucose homeostasis in diabetic patients and restore insulin sensitivity. The present invention contemplates these compounds may reduce blood glucose levels and diabetic serum marker hemoglobin A1c glycosylation level. The present invention provides kits comprising compounds or their pharmaceutical acceptable salts for administering to an animal or patients with symptoms of type II diabetes.

In an embodiment, the present invention provides a method of using ERRα modulators for treatment of type II diabetes.

In another embodiment, the present invention provides a method of using compounds with Formula I or their pharmaceutical acceptable salts for treatment of patients or animals with related diseases.

In another embodiment, the present invention provides a method of using compounds mentioned or their pharmaceutical acceptable salts for treatments of diseases related to ERRα including but not limited to: (1) Type II diabetes; (2) hyperglycemia; (3) reduced glucose tolerance; (4) insulin resistance; (5) obesity; (6) abnormal fat metabolism; (7) dyslipidemia; (8) hyperlipidemia; (9) hypertriglyceridemia; (10) hypercholesterolemia; (11) low levels of HDL; (12) high levels of LDL; (13) atherosclerosis; (14) vascular restenosis; (15) central obesity; (16) metabolic syndrome; (17) fatty liver.

In another embodiment, the present invention relates to compounds or their pharmaceutical acceptable salts for treatments of osteoporosis or related diseases.

In another embodiment, the present invention provides a method of using compounds mentioned or their pharmaceutical acceptable salts for treatments of dyslipidemia, hyperglycemia, atherosclerosis, low HDL levels, high LDL levels, hyperlipidemia, hypertriglyceridemia, etc.

The compound may be used alone or advantageously may be administered with a cholesterol biosynthesis inhibitor, particularly an HMG-CoA reductase inhibitor such as lovastatin, simvastatin, rosuvastatin, pravastatin, fluvastatin, atorvastatin, rivastatin, itavastatin, or ZD-4522. The compound may also be used advantageously in combination with other lipid lowering drugs such as cholesterol absorption inhibitors (for example stanol esters, sterol glycosides such as tiqueside, and azetidinones such as ezetimibe), ACAT inhibitors (such as avasimibe), CETP inhibitors, niacin, bile acid sequestrants, microsomal triglyceride transport inhibitors, and bile acid reuptake inhibitors. These combination treatments may also be effective for the treatment or control of one or more related conditions selected from the group consisting of hypercholesterolemia, atherosclerosis, hyperlipidemia, hypertriglyceridemia, dyslipidemia, high LDL, and low HDL.

Another aspect of the invention provides a method of treating inflammatory conditions, including inflammatory bowel disease, Crohn's disease, and ulcerative colitis by administering an effective amount of a compound of this invention to a patient in need of treatment. Additional inflammatory diseases that may be treated with the instant invention include gout, rheumatoid arthritis, osteoarthritis, multiple sclerosis, asthma, ARDS, psoriasis, vasculitis, ischemia/reperfusion injury, frostbite, and related diseases.

The compounds as defined herein may be used to treat diseases according to the following methods, as well as other diseases not listed below:
(1) A method for treating non-insulin dependent diabetes mellitus (type 2 diabetes) in a human or other mammalian patient in need of such treatment which comprises administering to the patient a therapeutically effective amount of a compound of Formula I;
(2) A method for treating or controlling hyperglycemia in a human or other mammalian patient in need of such treatment which comprises administering to the patient a therapeutically effective amount of a compound of Formula I;
(3) A method for treating or controlling the metabolic syndrome in a human or other mammalian patient in need of such treatment which comprises administering to the patient a therapeutically effective amount of a compound of Formula I;
(4) A method for treating or controlling obesity in a human or other mammalian patient in need of such treatment which comprises administering to the patient a therapeutically effective amount of a compound of Formula I;
(5) A method for treating or controlling hypercholesterolemia in a human or other mammalian patient in need of such treatment which comprises administering to the patient a therapeutically effective amount of a compound of Formula I;
(6) A method for treating or controlling hypertriglyceridemia in a human or other mammalian patient in need of such treatment which comprises administering to the patient a therapeutically effective amount of a compound of Formula I;
(7) A method for treating or controlling one or more lipid disorders, including mixed or diabetic dyslipidemia, low HDL cholesterol, high LDL cholesterol, hyperlipidemia, hypercholesterolemia, and hypertriglyceridemia in a human or other mammalian patient in need of such treatment which comprises administering to the patient a therapeutically effective amount of a compound of Formula I;
(8) A method for reducing the risks of adverse sequelae associated with metabolic syndrome in a human or other mammalian patient in need of such treatment which comprises administering to the patient a therapeutically effective amount of a compound of Formula I; and
(9) A method for treating atherosclerosis, for reducing the risk of developing atherosclerosis, for delaying the onset of atherosclerosis, and/or reducing the risk of sequelae of atherosclerosis in a human or other mammalian patient in need of such treatment or at risk of developing atherosclerosis or sequelae of atherosclerosis, which comprises administering to the patient a therapeutically effective amount of a compound of Formula I. Sequelae of atherosclerosis include for example angina, claudication, heart attack, stroke, etc.

### Administration and Dose Ranges

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of Formula I are administered orally.

The effective dosage of active ingredient employed may vary depending on the particular compound employed, the mode of administration, the condition being treated and the severity of the condition being treated. Such dosage may be ascertained readily by a person skilled in the art.

When treating or controlling diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 500 milligram per kilogram of animal body weight, preferably given as a single daily dose or in divided doses two to four times a day, or in sustained release form. For most large mammals, the total daily dosage is from about 0.1 milligrams to about 1500 milligrams, preferably from about 0.5 milligram to about 100 milligrams. In the case of a 70 kg adult human, the total daily dose will generally be from about 1 milligram to about 500 milligrams. For a particularly potent compound, the dosage for an adult human may be as low as 0.1 mg. The dosage regimen may be adjusted within this range or even outside of this range to provide the optimal therapeutic response.

Oral administration will usually be carried out using tablets. Examples of doses in tablets are 0.1mg, 0.2mg, 0.25mg, 0.5 mg, 1 mg, 2 mg, 5 mg, 10 mg, 25 mg, 50 mg, 100 mg, and 250 mg. Other oral forms can also have the same dosages (e.g. capsules).

### Pharmaceutical Compositions

Another aspect of the present invention provides pharmaceutical compositions which comprise a compound of Formula I and a pharmaceutically acceptable carrier. The pharmaceutical compositions of the present invention comprise a compound of Formula I or a pharmaceutically acceptable salt as an active ingredient, as well as a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids. A pharmaceutical composition may also comprise a prodrug, or a pharmaceutically acceptable salt thereof, if a prodrug is administered.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as coin starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula I may also be administered parenterally. Solutions or suspensions of these active compounds can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

### Metabolites-Prodrugs

Therapeutically active metabolites, where the metabolites themselves fall within the scope of the claimed invention, are also compounds of the current invention. Prodrugs, which are compounds that are converted to the claimed compounds as they are being administered to a patient or after they have been administered to a patient, are also compounds of this invention.

### Combination Therapy

Compounds of Formula I may be used in combination with other drugs that may also be useful in the treatment or amelioration of the diseases or conditions for which compounds of Formula I are useful. Such other drugs may be administered, by a route and in an amount commonly used therefore, contemporaneously or sequentially with a compound of Formula I. When a compound of Formula I is used contemporaneously with one or more other drugs, a pharmaceutical composition in unit dosage form containing such other drugs and the compound of Formula I is preferred. However, the combination therapy also includes therapies in which the compound of Formula I and one or more other drugs are administered on different overlapping schedules. It is also contemplated that when used in combination with one or more other active ingredients, the compound of the present invention and the other active ingredients may be used in lower doses than when each is used singly. Accordingly, the pharmaceutical compositions of the present invention include those that contain one or more other active ingredients, in addition to a compound of Formula I.

Examples of other active ingredients that may be administered in combination with a compound of Formula I, and either administered separately or in the same pharmaceutical composition, include, but are not limited to:
1) PPAR gamma agonists and partial agonists, including both glitazones and non-glitazones (e.g. troglitazone, pioglitazone, englitazone, MCC-555, rosiglitazone, balaglitazone, netoglitazone, T-131, LY-300512, and LY-818);
2) biguanides such as metformin and phenformin;
3) protein tyrosine phosphatase-IB (PTP-1B) inhibitors;
4) dipeptidyl peptidase IV (DP-IV) inhibitors;
5) insulin or insulin mimetics;
6) sulfonylureas such as tolbutamide and glipizide, or related materials;
7) α-glucosidase inhibitors (such as acarbose);
8) agents which improve a patient's lipid profile, such as (i) HMG-CoA reductase inhibitors (lovastatin, simvastatin, rosuvastatin, pravastatin, fluvastatin, atorvastatin, rivastatin, itavastatin, ZD-4522 and other statins), (ii) bile acid sequestrants (cholestyramine, colestipol, and dialkylaminoalkyl derivatives of a cross-linked dextran), (iii) nicotinyl alcohol, nicotinic acid or a salt thereof, (iv) PPARα agonists such as fenofibric acid derivatives (gemfibrozil, clofibrate, fenofibrate and bezafibrate), (v) cholesterol absorption inhibitors, such as for example ezetinibe, (vi) acyl CoA:cholesterol acyltransferase (ACAT) inhibitors, such as avasimibe, (vii) CETP inhibitors, and (viii) phenolic anti-oxidants, such as probucol;
9) PPARα/γ dual agonists, such as KRP-297, muraglitazar, tesaglitazar, farglitazar, and JT-501;
10) PPARα/γ agonists such as those disclosed in WO097/28149;
11) antiobesity compounds such as fenfluramine, dexfenfluramine, phentiramine, subitramine, orlistat, neuropeptide Y5 inhibitors, Mc4r agonists, cannabinoid receptor 1 (CB-1) antagonists/inverse agonists, and .beta.3 adrenergic receptor agonists;
12) ileal bile acid transporter inhibitors;
13) agents intended for use in inflammatory conditions such as aspirin, non-steroidal anti-inflammatory drugs, glucocorticoids, azulfidine, and cyclo-oxygenase 2 selective inhibitors;
14) glucagon receptor antagonists;
15) GLP-1 and its analogs, such as exenitide;,
16) GIP-1
17) GLP-1 receptor agonists.

The above combinations include combinations of a compound of the present invention not only with one other active compound, but also with two or more other active compounds. Non-limiting examples include combinations of compounds having Formula I with two or more active compounds selected from biguanides, sulfonylureas, HMG-CoA reductase inhibitors, other PPAR agonists, PTP-1B inhibitors, DP-IV inhibitors, and anti-obesity compounds.

### Example 1

### Preparation of (7-methoxy-3-methyl-2-phenylquinazolin-4(3H)-one)

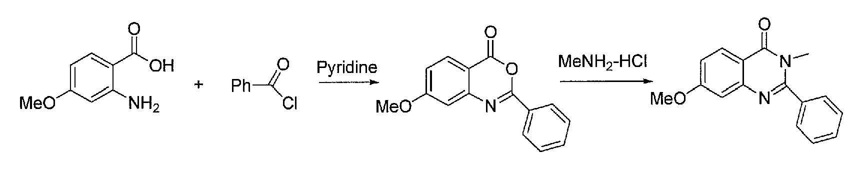

### Step1, preparation of (7-methoxy-2-phenyl -4H-benzo[d][1,3] oxazin -4-one)

The solution of 2-amino-4-methoxybenzoic acid (1.67 g, 10mmol) in pyridine (10mL) was added dropwisely the solution of benzoyl chloride (1.4g, 10mmol) in pyridine (5mL) at room temperature. The reaction was stirred for 6h at room temperature. Then the mixture was poured into ice-water (50g), extracted with ethyl acetate, dried over Na₂SO₄, and filtered. The filtrate was concentrated in vacuo and further purified by flash chromatography on silica gel (20% ethyl acetate/petroleum ether) to obtain the title compound (1.96g, 77.5%) as a white solid.

### Step2, preparation of (7-methoxy-3-methyl-2 -phenylquinazolin-4(3H)-one)

The compound obtained in the step 1 (0.253g, 1.0 mmol) and methylamine hydrochloride (0.675g ,10 mmol) were mixed in DMF (10 mL), and heated to reflux for 5h. Then the mixture was poured into ice-water (50g), extracted with ethyl acetate, dried over Na2SO4, and filtered. The filtrate was concentrated in vacuo and further purified by flash chromatography on silica gel (ethyl acetate: petroleum ether = 1:1) to obtain the title compound (0.14g, 51 %) as a white solid.
¹HNMR (400 MHz, CDCl₃), □ δ 8.25 (d, *J* = 8.8 Hz, 1H), 7.58 ∼ 7.25 (m, 5 H); 7.14 (d, *J* = 2.4 Hz, 1H), 7.8, (dd, *J* = 2.4, 8.8 Hz, 1H), 3.94 (s, 3 H), 3.48 (s, 3H);
**MS(ESI), m/z: 266 (M⁺).**

### Example 2

### Preparation of (2-(4-chlorophenyl)-7-methoxy-3-methylquinazolin-4(3H)-one)

**Synthetic method is the same as that of the Example 1.**
¹HNMR (400 MHz, CDCl₃), □ δ 8.21 (d, *J* = 8.4 Hz, 1H), 7.53 ∼ 7.10 (m, 4 H); 7.11 ∼ 7.06 (m, 2H), 3.91 (s, 3 H), 3.48 (s, 3H);
**MS(ESI), m/z: 300 (M⁺).**

### Example 3

### Preparation of (2-(4-fluorophenyl)-7-methoxy-3-methylquinazolin-4(3H)-one)

**Synthetic method is the same as that of the Example 1.**
¹HNMR (400 MHz, CDCl₃), □ δ 8.19 (d, *J* = 8.8 Hz, 1H), 7.59 ∼ 7.56 (m, 2 H); 7.26 ∼ 7.19 (m, 2 H), 7.10 (d, *J* = 2.0 Hz, 1H), 7.06 (dd, *J* = 2.0, 8.8 Hz, 1H), 3.89 (s, 3 H), 3.46 (s, 3H);
**MS(ESI), m/z: 285 (M+H)⁺.**

### Example 4

### Preparation of (2-(4-bromophenyl)-7-methoxy-3-methylquinazolin-4(3H)-one)

**Synthetic method is the same as that of the Example 1.**
¹HNMR (400 MHz, CDCl₃), □ δ 8.21 (d, *J* = 8.6 Hz, 1H), 7.60 (d, J = 6.8 Hz, 2 H); 7.45 (d, J = 6.8 Hz, 2 H), 7.11∼7.07 (m, 2 H), 3.91 (s, 3 H), 3.48 (s, 3H);
**MS(ESI), m/z: 345 (M)⁺.**

### Example 5

### Preparation of (2-(3-fluorophenyl)-7-methoxy-3-methylquinazolin-4(3H)-one)

**Synthetic method is the same as that of the Example 1.**
¹HNMR (400 MHz, CDCl₃), □ δ 8.23 (d, *J* = 8.8 Hz, 1H), 7.54 ∼ 7.49 (m, 2 H); 7.36 ∼ 7.21 (m, 2 H), 7.12 ∼ 7.08 (m, 2H), 3.91 (s, 3 H), 3.48 (s, 3H);
**MS(ESI), m/z: 284 (M)⁺.**

### Example 6

### Preparation of (2-(4-bromophenyl)-7-hydroxy-3-methylquinazolin-4(3H)-one)

**Synthetic method is the same as that of the Example 1.**
¹HNMR (400 MHz, DMSO-_{d6}), □ δ 10.52 (s, 1H), 8.01 (d, *J* = 8.8 Hz, 1H), 7.60 (d, *J* = 8.4 Hz, 2 H); 7.43 (d, *J* = 8.4 Hz, 2 H), 6.98 (dd, *J* = 2.4 8.8 Hz, 1 H), 6.91 (d, *J* = 2.4 Hz, 1 H), 3.32 (s, 3 H);
**MS(ESI), m/z: 331 (M)⁺, 333(M+2H)⁺.**

### Example 7

### Preparation of (7-methoxy-3-methyl-2-(pyridin-3-yl)quinazolin-4(3H)-one)

**Synthetic method is the same as that of the Example 1.**
¹HNMR (400 MHz, DMSO-_{d6}), □ δ 9.17 (d, J = 2.0 Hz, 1H), 8.75 (dd, *J* = 2.0, 4.8 Hz, 1H), 8.43 ∼ 8.40 (m, 1 H); 8.38 (d, *J* = 2.8 Hz, 1 H), 7.71 (d, *J* = 8.8 Hz, 1 H), 7.63 (dd, *J* = 5.0, 8.0 Hz, 1H), 6.75 (dd, *J* = 2.8, 8.8 Hz, 1 H), 3.89 (s, 3 H), 2.92 (s, 3 H);
**MS(ESI), m/z: 267 (M)⁺.**

### Example 8

### Preparation of (7-methoxy-3-methyl-2-(pyridin-4-yl)quinazolin-4(3H)-one)

**Synthetic method is the same as that of the Example 1.**
¹HNMR (400 MHz, CDCl₃), □ δ 8.75 (dd, *J* = 1.6,4.4 Hz, 2 H), 8.16 (d, J = 9.2 Hz, 1 H); 7.42 (dd, *J* = 1.6, 4.4 Hz, 2 H), 3.84 (s, 3 H), 3.41 (s, 3 H);
**MS(ESI), m/z: 268 (M+H)⁺.**

### Example 9

### Preparation of (3-methyl-2-phenylpyrido[2,3-d]pyrimidin-4(3H)-one)

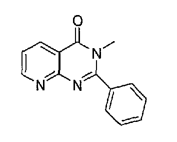

**Synthetic method is the same as that of the Example 1.**
¹HNMR (400 MHz, CDCl₃), □ δ 8.99 (dd, *J* = 1.6,4.4 Hz, 1 H), 8.66 (dd, J = 4.0, 8.0 Hz, 1 H); 7.65 (dd, *J* = 1.6, 7.0 Hz, 2 H), 7.54 (dd, *J* = 1.6, 7.0 Hz, 2 H), 7.53 (m, 1H), 7.45 (dd, *J* = 4.4, 8.0 Hz, 2 H), 3.56 (s, 3 H);
**MS(ESI), m/z: 236 (M)⁺- H⁺.**

### Example 10

### Preparation of (3-methyl-2-phenylpyrido[4,3-d]pyrimidin-4(3H)-one)

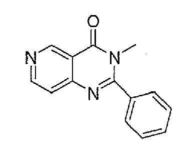

**Synthetic method is the same as that of the Example 1.**
**MS(ESI), m/z: 236 (M)⁺.**

### Example 11

### Preparation of (3-methyl-2-phenylquinazolin-4(3H)-one)

**Synthetic method is the same as that of the Example 1.**
¹HNMR (400 MHz, CDCl₃), □ δ 8.35 (d, *J* = 8.3 Hz, 1 H), 7.77 ∼ 7.74 (m, 2 H); 7.59 ∼ 7.49 (m, 6 H), 3.50 (s, 3 H);
MS(ESI), **m/z:** 235 (M)**⁺- H⁺,** 236 (M)**⁺.**

### Example 12

### Preparation of (7-hydroxy-3-methyl-2-phenylquinazolin-4(3H)-one)

**Compound 12 was synthesized by demethylation of compound in example 1 in the solution of HBr-HOAc.**
¹HNMR (400 MHz, DMSO-_{d6}), □ δ 8.01 (d, *J* = 8.4 Hz, 1 H), 7.63 (dd, J = 2.0, 7.5 Hz, 2 H); 7.54 ∼ 7.53 (m, 3H H), 6.99 (dd, J = 2.4, 8.3 Hz, 1H), 6.91 (d, J = 2.0 Hz, 1H), 3.17 (s, 3 H);
**MS(ESI), m/z: 251 (M)⁺- H⁺, 252 (M)⁺.**

### Example 13

### Preparation of (7-ethoxy-3-methyl-2-phenylquinazolin-4(3H)-one)

**Synthetic method is the same as that of the Example 1.**
¹HNMR (400 MHz, CDCl₃), □ δ 8.21 (d, *J* = 8.4 Hz, 1 H), 7.56 ∼ 7.51 (m, 5 H); 7.10 (d, J = 2.0 Hz, 1H), 7.06 (dd, J = 2.0, 8.8 Hz, 1 H), 4.10 (q, J = 7.2 Hz, 2 H), 3.47 (s, 3 H), 1.46 (t, J = 7.2 Hz, 3H);
MS(ESI), m/z: 279 (M)⁺- H⁺, 280 (M)⁺.

### Example 14

### Preparation of (7-isopropoxy-3-methyl-2-phenylquinazolin-4(3H)-one)

Synthetic method is the same as that of the Example 1.
¹HNMR (400 MHz, CDCl₃), □ δ 8.20 (d, *J* = 8.8Hz, 1 H), 7.55 ∼ 7.51 (m, 5 H); 7.11 (s, 1H), 7.03 (d, J = 8.8 Hz, 1 H), 4.7 (m, 1 H), 3.46 (s, 3 H), 1.39 (d, J = 6 Hz, 6H);
**MS(ESI), m/z: 294 (M)⁺.**

### Example 15

### Preparation of (2-(3-chlorophenyl)-7-methoxy-3-methylquinazolin-4(3H)-one)

**Synthetic method is the same as that of the Example 1.**
¹HNMR (400 MHz, CDCl₃), □ δ 8.18(d, J = 8.6 Hz, 1 H), 7.53 (s, 1 H); 7.48 ∼ 7.35 (m, 3 H), 7.08 ∼ 7.00 (m, 2H), 3.84 (s, 3 H), 3.41 (s, 3 H);
**MS(ESI), m/z: 301 (M+H)⁺, 303 (M+3H)⁺.**

### Example 16

### Preparation of (7-methoxy-3-methyl-2-(naphthalen-1-yl)quinazolin-4(3H)-one)

**Synthetic method is the same as that of the Example 1.**
¹HNMR (400 MHz, CDCl₃), □ δ 8.31 (d, J = 8.8 Hz, 1 H), 8.01 (dd, J = 4.4, 9.0 Hz, 1H), 7.95 (d, J = 8.4 Hz, 1H), 7.62 ∼ 7.50 (m, 5 H), 7.17 (d, J = 2.4Hz, 1H), 7.13 (dd, J = 2.4, 8.8 Hz, 1H), 3.90 (s, 3 H), 3.29 (s, 3 H);
**MS(ESI), m/z: 317 (M+H)⁺, 318 (M+2H)⁺.**

### Example 17

### Preparation of (7-methoxy-3-(2-morpholinoethyl)-2-phenylquinazolin-4(3H)-one)

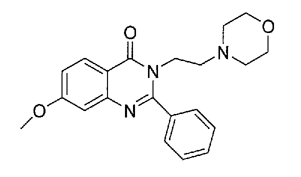

**Synthetic method is the same as that of the Example 1.**
¹HNMR (400 MHz, COCl₃), □ δ 8.21 (d, J = 8.8 Hz, 1 H), 7.56 ∼ 7.49 (m, 5 H), 7.11 (d, J = 2.4 Hz, 1H), 7.06 (dd, J = 2.4, 8.8 Hz, 1H), 4.13 (t, J = 6.8 Hz, 2H), 3.89 (s, 3 H), 3.58 (t, J = 4.8 Hz, 4 H), 2.52 (t, J = 6.8 Hz, 2H), 2.25 (t, J = 4.8 Hz, 4H);
**MS(ESI), m/z: 366 (M+H)⁺.**

### Example 18

### Preparation of (2-(4-fluorophenyl)-7-methoxy-3-(2-morpholinoethyl) quinazolin-4(3H)-one)

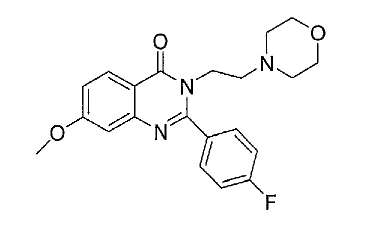

**Synthetic method is the same as that of the Example 1.**
¹HNMR (400 MHz, CDCl₃), □ δ 8.20 (d, J = 8.8 Hz, 1 H), 7.59 ∼ 7.55 (m, 2 H), 7.26 ∼ 7.19 (m, 2 H), 7.11 ∼ 7.07 (m, 2 H) 4.14 (t, J = 6.8 Hz, 2H), 3.90 (s, 3 H), 3.56 (t, J = 4.8 Hz, 4 H), 2.53 (t, J = 6.8 Hz, 2H), 2.25 (t, J = 4.8 Hz, 4H);
**MS(ESI), m/z: 384 (M+H)⁺.**

### Example 19

### Preparation of (2-(4-chlorophenyl)-7-methoxy-3-(2-morpholinoethyl) quinazolin-4(3H)-one)

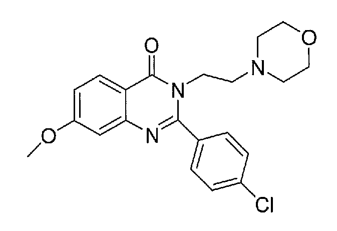

**Synthetic method is the same as that of the Example 1.**
¹HNMR (400 MHz, CDCl₃), □ δ 8.20 (d, J = 8.8 Hz, 1 H), 7.54 ∼ 7.49 (m, 4 H), 7.08 (d, J = 8.8 Hz, 1 H), 7.68 (s, 1 H) 4.14 (t, J = 6.8 Hz, 2H), 3.90 (s, 3 H), 3.57 (t, J = 4.8 Hz, 4 H), 2.54 (t, J = 6.8 Hz, 2H), 2.28 (t, J = 4.8 Hz, 4H);
**MS(ESI), m/z: 400 (M+H)⁺, 402 (M+3H)⁺.**

### Example 20

### Preparation of (7-chloro-3-methyl-2-phenylquinazolin-4(3H)-one)

**Synthetic method is the same as that of the Example 1.**
¹HNMR (400 MHz, CDCl₃), □ δ 8.19 (d, J = 8.4 Hz, 1 H), 7.67 (d, J = 2.0 Hz, 1 H), 7.51 ∼ 7.46 (m, 5H), 7.38 (dd, J = 2.0, 8.8 Hz, 1H), 3.43 (s, 3 H);
**MS(ESI), m/z: 271 (M+H)⁺, 273 (M+3H)⁺.**

### Example 21

### Preparation of (7-chloro-3-(2-morpholinoethyl)-2-phenylquinazolin-4(3H)-one)

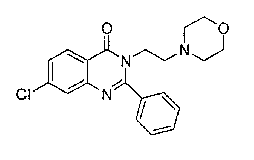

**Synthetic method is the same as that of the Example 1.**
¹HNMR (400 MHz, CDCl₃), □ δ 8.24 (d, J = 8.4 Hz, 1 H), 7.72 (d, J = 2.0 Hz, 1 H), 7.57 ∼ 7551 (m, 5 H), 7.44 (dd, J = 2.0, 8.4 Hz, 1 H), 4.17 (t, J = 6.8 Hz, 2 H), 3.53 (t, J = 4.8 Hz, 4 H), 2.52 (t, J = 6.8 Hz, 2 H), 2.21 (t, J = 4.8 Hz, 4 H);
**MS(ESI), m/z: 370 (M+H)⁺, 373 (M+3H)⁺.**

### Example 22

### Preparation of (7-chloro-2-(4-chlorophenyl)-3-methylquinazolin-4(3H)-one)

**Synthetic method is the same as that of the Example 1.**
¹HNMR (400 MHz, CDCl₃), □ δ8.18 (d, J = 8.8 Hz, 1 H), 7.65 (d, J = 2.0 Hz, 1 H), 7.50 ∼ 7.46 (m, 4H), 7.39 (dd, J = 2.0, 8.4 Hz, 1H), 3.43 (s, 3 H);
MS(ESI), m/z: 305 (M)⁺.

### Example 23

### Preparation of (7-chloro-2-(4-fluorophenyl)-3-methylquinazolin-4(3H)-one)

**Synthetic method is the same as that of the Example 1.**
¹HNMR (400 MHz, CDCl₃), □ δ8.28 (d, J = 8.8 Hz, 1 H), 7.72 (s, 1 H), 7.65 ∼ 7.55 (m, 2 H), 7.45 (d, J = 8.8 Hz, 1H), 7.25 ∼ 7.20 (m, 2 H), 3.50 (s, 3 H);
**MS(ESI), m/z: 288 (M)⁺, 287 (M⁺ - H⁺).**

### Example 24

### Preparation of (6-methoxy-3-methyl-2-phenylquinazolin-4(3H)-one)

**Synthetic method is the same as that of the Example 1.**
¹HNMR (400 MHz, CDCl₃), □ δ7.69 (s, 1 H), 7.68 (d, J = 8.8 Hz, 1 H), 7.58 ∼ 7.46 (m, 4 H), 7.39 ∼ 7.34 (m, 2 H), 4.00 (s, 3H), 3.54 (s, 3 H);
MS(ESI), m/z: 266 (M)⁺, 265 (M⁺ - H⁺).

### Example 25

### Preparation of (2-(4-chlorophenyl)-6-methoxy-3-methylquinazolin-4(3H)-one)

Synthetic method is the same as that of the Example 1.
¹HNMR (400 MHz, CDCl₃), □ δ7.68 (d, J = 2.8 Hz, 1 H), 7.66 (d, J = 8.8 Hz, 1 H), 7.54 ∼ 7.49 (m, 4 H), 7.37 (dd, J = 2.8, 8.8 Hz, 1 H), 3.95 (s, 3H), 3.51 (s, 3 H);
**MS(ESI), m/z: 301 ( M⁺ + H⁺), 303 (M⁺ + 3H⁺).**

### Example 26

### Preparation of (2-(4-fluorophenyl)-6-methoxy-3-methylquinazolin-4(3H)-one)

**Synthetic method is the same as that of the Example 1.**
¹HNMR (400 MHz, CDCl₃), □ δ7.62 (d, J = 2.8 Hz, 1 H), 7.59 (d, J = 8.8 Hz, 1 H), 7.52 ∼ 7.49 (m, 2 H), 7.29 (dd, J = 2.8, 8.8 Hz, 1 H), 7.19 ∼ 7.13 (m, 2 H), 3.88 (s, 3H), 3.44 (s, 3 H);
**MS(ESI), m/z: 284 ( M⁺).**

### Example 27

### Preparation of (6-iodo-7-methoxy-3-methyl-2-phenylquinazolin-4(3H)-one)

**Synthetic method is the same as that of the Example 1.**
¹HNMR (400 MHz, CDCl₃), □ δ8.68 (s, 1 H), 7.50 ∼ 7.45 (m, 5 H), 7.29 (dd, J = 2.8, 8.8 Hz, 1 H), 7.19 (s, 1 H), 3.92 (s, 3H), 3.41 (s, 3 H);
**MS(ESI), m/z: 392 ( M⁺).**

### Example 28

### Preparation of ( 3,4-dihydro-7-methoxy-3-methyl-N-(2-morpholinoethyl)-4-oxo-2-phenylquinazoline-6-carhoxami de)

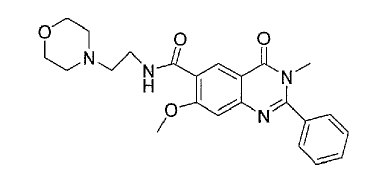

**Compound 28 was synthesized by carbonylation of compound 27.**
¹HNMR (400 MHz, CDCl₃), □ δ9.08 (s, 1 H), 8.33 (s, 1 H), 7.58 -7.55 (m, 5 H), 7.19 (s, 1 H), 4.11 ∼ 4.07 (m, 2 H), 4.08 (s, 3H), 3.83 (m, 4 H), 3.74 ∼ 3.71 (m, 2H), 3.50 (s, 3 H), 2.93 ∼ 2.75 (m, 4 H);
**MS(ESI), m/z: 423 ( M⁺ + H⁺).**

### Example 29

### Preparation of (6-hydroxy-7-methoxy-3-methyl-2-phenylquinazolin-4(3H)-one)

**Synthetic method is the same as that of the Example 1.**
¹HNMR (400 MHz, CDCl₃), □ δ7.76 (s, 1 H), 7.52 (m, 6 H), 7.16 (s, 1 H), 4.00 (s, 3H), 3.48 (s, 3 H);
**MS(ESI), m/z: 281 (M⁺ - H⁺).**

### Example 30

### Preparation of (6-(2-morpholinoethoxy)-7-methoxy-3-methyl-2-phenylquinazolin-4(3H)-one )

**Compound 30 was synthesized from compound 29.**
¹HNMR (400 MHz, CDCl₃), □ δ7.66 (s, 1 H), 7.54 ∼ 7.50 (m, 5 H), 7.15 (s, 1 H), 4.25 ∼ 4.20 (m, 2 H), 3.95 (s, 3H), 3.73 (t, J = 4.4 Hz, 4 H), 3.48 (s, 3 H), 2.56 (t, J = 7.2 Hz, 2 H), 2.50 ∼ 2.43 (m, 4 H);
**MS(ESI), m/z: 396 (M⁺ + H⁺).**

### Example 31

### Preparation of (2-cyclohexyl-7-methoxy-3-methylquinazolin-4(3H)-one)

**Synthetic method is the same as that of the Example 1.**
¹HNMR (400 MHz, CDCl₃), □ δ8.12 (d, J = 8.8 Hz, 1 H), 7.00 (s, 1 H), 6.98 (d, J = 8.8 Hz, 1 H), 3.90 (s, 3H), 3.63 (s, 3 H), 2.79 (t, J = 11.6 Hz, 1 H), 2.03 ∼ 1.70 (m, 6 H), 1.45 ∼ 1.25 (m, 4 H);
**MS(ESI), m/z: 273 (M⁺ + H⁺).**

### Example 32

### Preparation of (2-isopropyl-7-methoxy-3-methylquinazolin-4(3H)-one)

**Synthetic method is the same as that of the Example 1.**
¹HNMR (400 MHz, COCl₃), □ δ8.14 (d, J = 8.8 Hz, 1 H), 7.00 (s, 1 H), 6.98 (d, J = 8.8 Hz, 1 H), 3.92 (s, 3H), 3.65 (s, 3 H), 3.20 (m, 1 H), 1.38 (d, J = 7.2 Hz, 6 H);
**MS(ESI), m/z: 232 (M⁺).**

### Example 33

### Preparation of (2,3-dimethyl-7-methoxyquinazo)in-4(3H)-one)

**Synthetic method is the same as that of the Example 1.**
¹HNMR (400 MHz, CDCl₃), □ δ8.12 (d, J = 8.8 Hz, 1 H), 7.00 ∼ 6.98 (m, 2 H), 3.88 (s, 3H), 3.58 (s, 3 H), 2.59 (s, 3 H);
**MS(ESI), m/z: 204 (M⁺).**

### Example 34

### Preparation of (2-tertbutyl-7-methoxy-3-methylquinazolin-4(3H)-one)

**Synthetic method is the same as that of the Example 1.**
¹HNMR (400 MHz, CDCl₃), □ δ8.13 (d, J = 8.8 Hz, 1 H), 7.01 (s, 1 H), 6.99 (d, J = 8.8 Hz, 1 H), 3.92 (s, 3H), 3.73 (s, 3 H), 1.55 (s, 9 H);
**MS(ESI), m/z: 246 (M⁺).**

### Example 35

This example illustrates that the compounds mentioned in this invention (such as compound 1, also named DK3, 7-methoxy-3-methyl-2-phenylquinazolin-4(3H)-one) and other compounds with core structure of formula I such as compound 4, also named DK6, 2-(4-bromophenyl)-7-methoxy-3-methylquinazolin-4(3H)-one, and compound 3, also named DK7, 2-(4-fluorophenyl)-7-methoxy-3-methylquinazolin-4(3H)-one, can effectively enhance the expression of reporter genes modulated by ERRα in HeLa cell, therefore these compounds can effectively enhance the function of ERRα.

To test the effect of the compounds on ERRα and other nuclear hormone receptors, HeLa cells were transiently transfected with expression vectors for the receptors along with appropriate reporter constructs according to methods known in the art. Suitable reporter gene constructs are well known to skilled workers in the fields of biochemistry and molecular biology. Other vectors known in the art can be used in the methods of the present invention.

GAL4 fusions containing receptor ligand binding domain fragments were constructed by fusing human ERRalpha, human ERRbeta and murine ERRgamma ligand binding domain sequences to the C-terminal end of the yeast GAL4 DNA binding domain (amino acids 1-147 accession X85976) to form the expression vectors GAL-hERRα, GAL-L-hERRβ and Gal-mERRγ, respectively pGAL is a control containing the yeast GAL4 DNA binding domain without receptor sequences. CMV-PGC-1α contains and expressed the PGC-1α coding sequences derived from PGC-1α (accession NM.sub.--008904).

HeLa cells for the activation assays were grown in Dulbecco's modified Eagle's medium supplemented with 10% resin charcoal-stripped fetal bovine serum at 37°C in 5% CO₂, One day prior to transfection, cells were plated to 50-80% confluence using phenol red free DMEM-FBS. The cells were transiently transfected by lipofection but other methods of transfection of DNA into cells can be utilized without deviating from the spirit of the invention. Luciferase reporter construct UASgx4-TK-Luc and cytomegalovirus-driven expression vector p-GAL, GAL-hERRα, GAL-L-hERRβ or Gal-mERRγ were added with CMVPGC-1α. The cells were treated for approximately 24 hours with phenol red free DMEM-FBS containing 0.01% DMSO (control) or 0.01% DMSO with increasing concentrations of DK compounds.

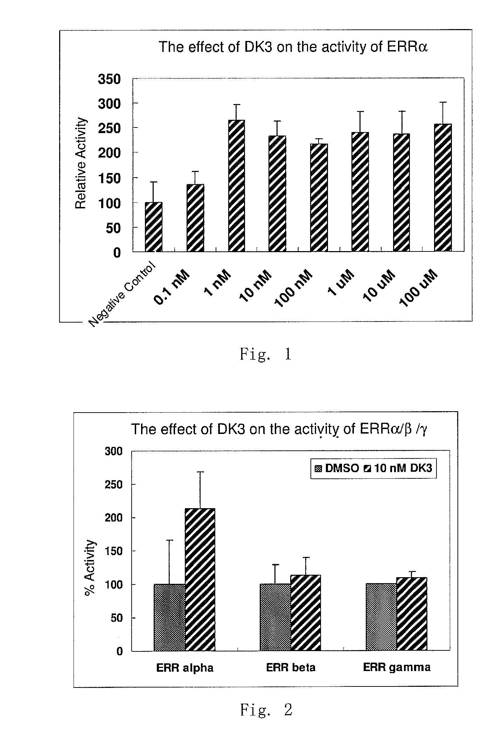

Compound DK3 dose-dependently enhances the activity of GAL-hERRα on reporter construct UASgx4-TK-Luc in the presence of CMV-PGC-1α. The EC₅₀ is estimated to be about 0.5 nM in HeLa cells (referring to Figs. 1, 2, 3). In addition, DK3 also dose-dependently reverses the suppressive effect of an ERRα specific inverse agonist XCT-790 (FIG. 2). Variations of triplicate measurements are indicated.

### Example 36

This example illustrates that compounds mentioned in this invention such as DK3 can effectively enhance the expression of PGC1α-promoter reporter gene in HeLa cell.

HeLa were transiently transfected with the pGL3-promoter (Promega) derivative pGL3-PGC1α-promoter and expression vector for ERRα. The Renilla-Luciferase pRL-CMV Vector (Promega) was included as a control for transfection efficiency. The full length human ERRα was cloned into the expression vector pCMV. The pGL3-PGC1α-promoter was generated by cloning an insert derived from a PCR reaction using human genomic DNA as template and primers based on the 2.6 kbp upstream sequence of the PGC1α transcriptional start site.

HeLa cells for the activation assays were grown in Dulbecco's modified Eagle's medium supplemented with 10% fetal bovine serum at 37°C in 5% CO₂. One day prior to transfection, cells were plated to 50-80% confluence using DMEM-FBS. The cells were transiently transfected by lipofection but other methods of transfection of DNA into cells can be utilized without deviating from the spirit of the invention. Luciferase reporter construct pGL3-PGC1α-promoter and cytomegalovirus-driven expression vector pCMV or pCMV -hERRα were added. The cells were treated for approximately 24 hours with DMEM-FBS containing 0.01 % DMSO (control) or 0.01 % DMSO with increasing concentrations of DK compounds.

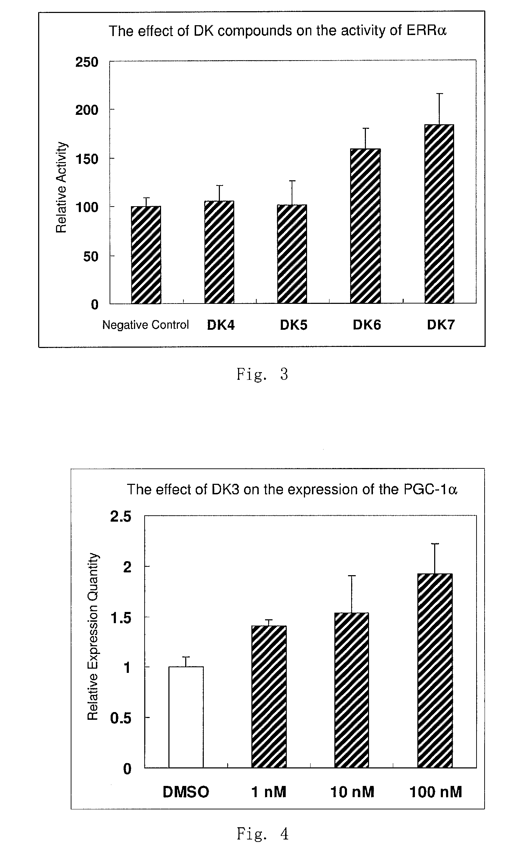

A dose-dependent enhancement of the PGC1α-promoter reporter gene driven by ERRα was observed for the compound DK3 (referring to FIG. 4). Variations of triplicate measurements are indicated.

### Example 37

Current example illustrates that compounds mentioned in this invention such as DK3 can effectively enhance the insulin-dependent glucose uptake in myoblasts L6 cell.

Rat myoblasts L6 were grown in differentiation medium (DMEM + 2% FBS) for 6 days to induce myotube formation. 0.01% DMSO (control) or 0.01% DMSO with either positive control rosiglitazone or DK1 was added to myotubes for 48 hrs. Cells were washed three times with 1 ml per well pre-warmed Phosphate Buffer Saline (PBS). 100 nM insulin diluted in 250 µl per well pre-warmed FCB buffer were added and incubated at 37°C for 20 minutes. 25 µl per well of 2DOG mixture prepared by combining 8.2 ml of PBS with 1.0 ml 11 mM glucose and 0.83 ml ³H-2-Deoxy-Glucose was added for 10 minutes. The reactions were stopped by adding ice-cold PBS and washed four times in ice-cold PBS. The reactions were than left to dry and scintillation solution were added to each well and transferred to tubes for measurement. (FCB buffer = Kreb's Ringer with Hepes 125 mM NaCl, 5 mM KCI, 1.8 mM CaCl₂, 2.6 mM MgSO₄ and 25 mM Hepes plus 2 mM Glucose and 0.3% BSA)

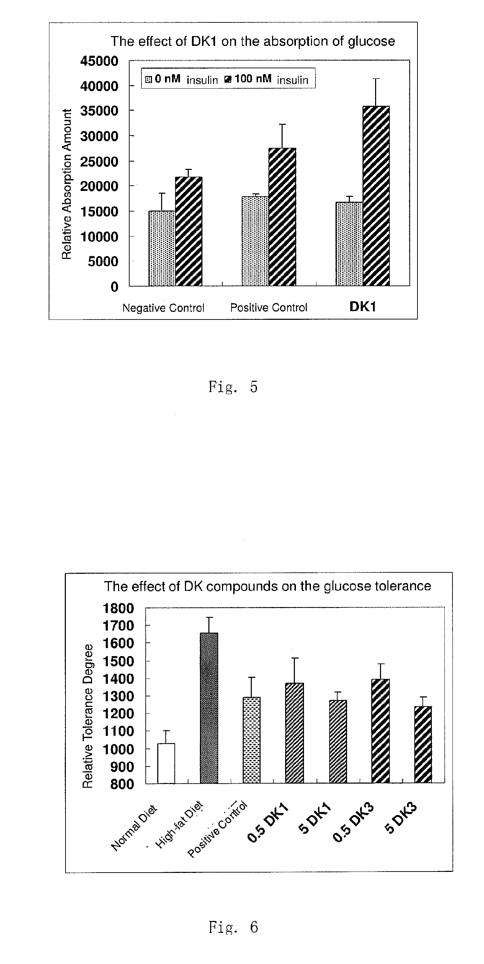

Insulin induces the ability of myotube to take up radioactive glucose. Anti-diabetic drug rosiglitazone enhances the uptake of glucose. DK1 enhances the insulin-dependent glucose uptake in myotubes (referring to FIG. 5) comparable to positive control rosigliatzone.

### Example 38

This example illustrates that compounds mentioned in this invention such as DK3 can effectively improve glucose tolerance in high-fat-diet mice.

Seven weeks old male C57BL/J6 mice were either fed chow diet or a high-fat-diet with 60% calories from lard for 10 weeks. Compounds were administered to animals by gavage for two weeks at different doses. Six groups of animals (n=5) were administered with either vehicle, 10 mg/kg/day rosiglitazone, 0.5 mg/kg/day DK1, 5 mg/kg/day DK1, 0.5 mg/kg/day DK3, or 5 mg/kg/day DK3. Animals were then orally fed glucose. Blood samples were withdrawn at time 0, 15, 30, 60 and 120 min. Blood glucose level was measured by monitor (Accu-chek Advantage, Roche). The changes in blood glucose level were plotted against time and the areas under the curve were calculated for the different groups.

Compared to positive control rosiglitazone given at 10 mg/kg/day, both DK1 and DK3 at either 0.5 or 5 mg/kg/day reduced the area under the curve of the oral glucose tolerance test (referring to FIG. 6), indicating that ERRα agonists DK1 and DK3 improve glucose tolerance *in vivo.*

### Example 39

Current example illustrates that compounds mentioned in this invention such as DK3 can effectively reduce the extent of fatty liver induced by high-fat-diet.

Seven weeks old male C57BL/J6 mice were either fed chow diet or a high-fat-diet with 60% calories from lard for 10 weeks. Compounds were administered to animals by gavage for two weeks at different doses. Six groups of animals (n=5) were administered with either vehicle, 10 mg/kg/day rosiglitazone, 0.5 mg/kg/day DK1, 5 mg/kg/day DK1, 0.5 mg/kg/day DK3, or 5 mg/kg/day DK3. Total body weight and livers wet weight from animals were measured. Liver samples from animals were prepared by histological methods.

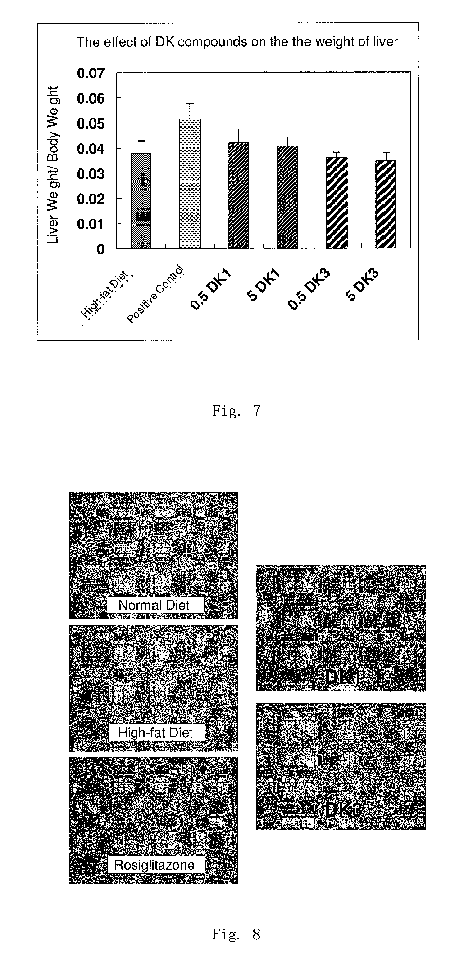

Compared to rosiglitazone given at 10 mg/kg/d, both DK1 and DK3 at either 0.5 or 5 mg/kg/day did not increase liver weight. In addition, both ERRα agonists DK1 and DK3 reduce the extent of fatty liver induced by high-fat-diet , as shown in Figs. 7 and 8.

## Claims

1. Compounds having formula I or their pharmaceutical acceptable salts or stereo isomers: 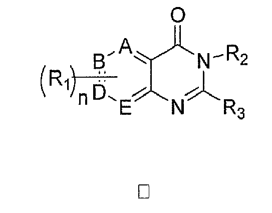 wherein:
A, B, D, and E are independently selected as CH or N;
n is 0, 1 or 2;
in the following, a is 0 or 1, b is 0 or 1;R₁ is selected from:
1) H;
2) halide;
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₇;
16) SO₂C₁∼C₈ alkyl;
17) (C=O)ₐO_{b}C₀∼C₈-NR₆R₅;
the alkyl, aryl, alkenyl, alkynyl, cycloalkyl and heterocycle group mentioned above can be substituted by 0, 1 or more than 1 substituted group independently selected from R₄;
R₂ is selected from:
1) H;
2) C₁∼C₈ alkyl:
3) aryl:
4) C₃∼C₈ alkenyl;
5) C₃∼C₈ alkynyl;
6) C₁~C₈ fluoroalkyl;
7) C₁∼C₆ aryl alkyl;
8) C₃∼C₆ cycloalkyl;
9) Heterocycle;
the alkyl, aryl, alkenyl, alkynyl, cycloalkyl and heterocycle group mentioned above can be substituted by 0, 1 or more than 1 substituted group independently selected from R₄;
R₃ is selected from:
1) (C=O)ₐO_{b}C₁∼C₈ alkyl;
2) (C=O)ₐO_{b} aryl;
3) (C=O)ₐO_{b}C₂∼C₈ alkenyl;
4) (C=O)ₐO_{b}C₂∼C₈ alkynyl;
5) O_{b}C₁∼C₈ fluoroalkyl;
6) (C=O)ₐNR₆R₅;
7) (C=O)ₐO_{b}C₃∼C₈ cycloalkyl;
8) (C=O)ₐO_{b} heterocycle;
9) (C=O)ₐO_{b}C₃∼C₆ cycloalkyl;
the alkyl, aryl, alkenyl, alkynyl, cycloalkyl and heterocycle group mentioned above can be substituted by 0, 1 or more than 1 substituted group independently selected from R₄;
R₄ is selected from:
1) H;
2) Halide;
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₇;
16) SO₂C₁∼C₈ alkyl;
the alkyl, aryl, alkenyl, alkynyl, cycloalkyl and heterocycle group mentioned above can be substituted by no more than 3 substituted group independently selected from R₅, OH, (C₁∼C₆) alkoxy, halide, COOH, CN, O(C=O)C₁∼C₆ alkyl, or NR₅R₆ groups;
R₅ and R₆ are independently selected from:
1) H;
2) (C=O)ₐO_{b}C₁∼C₈ alkyl;
3) (C=O)ₐO_{b} aryl;
4) (C=O)ₐO_{b}C₂∼C₈ alkenyl;
5) (C=O)ₐO_{b}C₂∼C₈ alkynyl;
6) O_{b}C₁∼C₈ fluoroalkyl;
7) (C=O)ₐO_{b}C₃∼C₈ cycloalkyl;
8) (C=O)ₐO_{b} heterocycle;
9) SO₂C₁∼C₈ alkyl;
10) (C=O)ₐO_{b}C₀∼C₈-NR₆R₅;
the alkyl, aryl, alkenyl, alkynyl, cycloalkyl and heterocycle group mentioned above can be substituted by no more than 3 substituted group independently selected from R_{6;} R₆ and R₅ may form a 4∼7-atom ring, or a bicyclic ring in which each ring is formed by 4∼7-atoms; the ring or bicyclic ring contains 1, 2, or 3 hetero atoms selected from N, O or S atom; the ring or bicyclic ring can be substituted by one or more substituted groups independently selected from R₅.

2. The compounds or their pharmaceutical acceptable salts or stereo isomers according to claim 1, wherein the compounds have formula II: 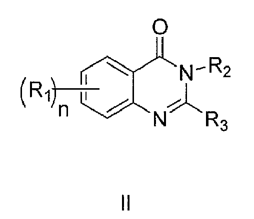 wherein, R₁∼R₃ and n are defined as that in claim 1.

3. The compounds or their pharmaceutical acceptable salts or stereo isomers according to claim 1, wherein the compounds have formula III∼VI: 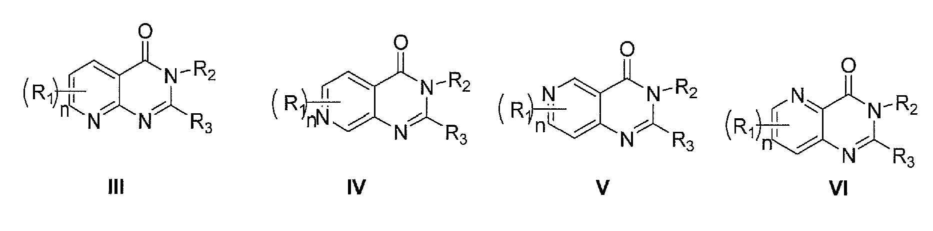 wherein, R₁∼R₃ and n are defined as that in claim 1.

4. The compounds or their pharmaceutical acceptable salts or stereo isomers according to claim 2, wherein the compounds have formula VII: 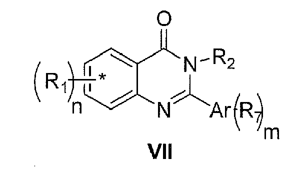 wherein
a is 0 or 1; b is 0 or 1;
m, n are independently selected as 0, 1, or 2;
Ar is an aromatic ring containing or without containing hetero atoms such as N, S or O;
R₁ and R₇ are independently selected from:
1) H;
2) Halide;
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₅;
16) SO₂C₁∼C₈ alkyl;
17) (C=O)ₐO_{b}C₀∼C₈-NR₆R₅;
the alkyl, aryl, alkenyl, alkynyl, cycloalkyl or heterocycle group mentioned above can be substituted by 0, 1 or more than 1 substituted group independently selected from R₄;
R₂ is selected from:
1) H;
2) C₁∼C₈ alkyl;
3) Aryl;
4) C₃∼C₈ alkenyl;
5) C₃∼C₈ alkynyl;
6) C₁∼C₈ fluoroalkyl;
7) C₁∼C₆ aryl alkyl;
8) C₃∼C₆ cycloalkyl;
9) Heterocycle;
the alkyl, aryl, alkenyl, alkynyl, cycloalkyl or heterocycle group mentioned above can be substituted by 1 or more than 1 substituted group independently selected from R₄;
R₄ is selected from:
1) H;
2) Halide;
3) OH;
4) NO₂;
5) CO₂H;
6) (C=O)ₐO_{b}C₁∼C₈ alkyl;
7) (C=O)ₐO_{b} aryl;
8) (C=O)ₐO_{b}C₂∼C₈ alkenyl;
9) (C=O)ₐO_{b}C₂∼C₈ alkynyl;
10) O_{b}C₁∼C₈ fluoroalkyl;
11) (C=O)ₐNR₆R₅;
12) CN;
13) (C=O)ₐO_{b}C₃∼C₈ cycloalkyl;
14) (C=O)ₐO_{b} heterocycle;
15) SO₂NR₆R₅;
16) SO₂C₁∼C₈ alkyl;
the alkyl, aryl, alkenyl, alkynyl, cycloalkyl or heterocycle group mentioned above can be substituted by no more than 3 substituted group independently selected from R₅, OH, (C₁∼C₆) alkoxy, halide, COOH, CN, O(C=O)C₁∼C₆ alkyl, or NR₅R₆ groups;
R₅ and R₆ are independently selected from:
1) H;
2) (C=O)ₐO_{b}C₁∼C₈ alkyl;
3) (C=O)ₐO_{b} aryl;
4) (C=O)ₐO_{b}C₂∼C₈ alkenyl;
5) (C=O)ₐO_{b}C₂∼C₈ alkynyl;
6) O_{b}C₁∼C₈ fluoroalkyl;
7) (C=O)ₐO_{b}C₃∼C₈ cycloalkyl;
8) (C=O)ₐO_{b} heterocycle;
9) SO₂C₁∼C₈ alkyl;
10) (C=O)ₐO_{b}C₀∼C₈-NR₆R₅;
the alkyl, aryl, alkenyl, alkynyl, cycloalkyl or heterocycle group mentioned above can be substituted by no more than 3 substituted group independently selected from R_{6.} R₆ and R₅ may form a 4∼7-atom ring, or a bicyclic ring in which each ring is formed by 4∼7-atoms; the ring or bicyclic ring contains 1, 2, or 3 hetero atoms selected from N, O or S atom; the ring or bicyclic ring can be substituted by one or more substituted groups independently selected from R_{5.}

5. The compounds or their pharmaceutical acceptable salts or stereo isomers according to claim 4, wherein the compounds have formula VIII: 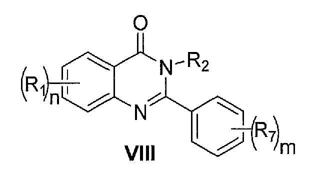 wherein R₁, R₂, R₇, m and n are defined the same as that of claim 4.

6. The compounds or their pharmaceutically acceptable salts or stereo isomers according to claim 5, wherein the compounds are:
7-methoxyl-3-methyl-2-phenyl-quinazolin-4(3H)-2-one; or
7-methoxyl-3-methyl-2-(4-bromophenyl)-quinazolin-4(3H)-2-one; or
7-methoxyl-3-methyl-2-(4-chlorophenyl)-quinazolin-4(3H)-2-one; or
7-methoxyl-3-methyl-2-(4-fluorophenyl)-quinazolin-4(3H)-2-one.

7. A pharmaceutical composition containing any one of the compounds or their pharmaceutically acceptable salts or stereo isomers according to any one of claims 1-6 and pro-drugs thereof.

8. Use of the compounds and their pharmaceutical acceptable salts or stereo isomers according to any one of claims 1-6 which function as modulators of estrogen-related receptors in manufacturing pharmaceutical composition f for the treatment of metabolic diseases.

9. The use of claim 8, wherein the metabolic diseases comprise: (1) Type II diabetes; (2) hyperglycemia; (3) reduced glucose tolerance; (4) insulin resistance; (5) obesity; (6) abnormal fat metabolism; (7) dyslipidemia; (8) hyperlipidemia; (9) hypertriglyceridemia; (10) hypercholesterolemia; (11) low levels of HDL; (12) high levels of LDL; (13) atherosclerosis; (14) vascular restenosis; (15) central obesity; (16) metabolic syndrome; (17) fatty liver.
